# EUROPEAN PATENT SPECIFICATION

(11) **EP 1 328 279 B1**
(45) Date of publication and mention of the grant of the patent: **26.08.2009**
(21) Application number: 01975298.9
(22) Date of filing: 20.09.2001
(51) Int. Cl.: A61K 31/70, A61F 2/02, A61P 35/00, A61P 9/10

(54) **COMPOSITIONS COMPRISING LIGNIN AND METHODS OF MAKING AND USING THE SAME**
ZUSAMMENSETZUNGEN AUS LIGNIN UND VERFAHREN ZU IHRER HERSTELLUNG UND VERWENDUNG
COMPOSITIONS COMPRENANT DE LA LIGNINE ET PROCEDES DE FABRICATION ET D'UTILISATION DE CELLES-CI

(30) Priority: 20.09.2000 US 665837
(43) Date of publication of application: 23.07.2003
(73) Proprietor: Aquamed Technologies, Inc., Sacremento, CA 95815 (US)
(72) Inventor: ALIMI, Hojabr, Petaluma, CA 94954 (US)
(74) Representative: Baldock, Sharon Claire
(86) International application number: PCT/US2001/029661
(87) International publication number: WO 2002/024208

(56) References cited:
- JP-A- 3 206 043
- US-A- 6 037 332
- SORIMACHI K ET AL: "Delayed cytocidal effects of lignin derivatives on virally transformed rat fibroblasts" CANCER DETECTION AND PREVENTION, vol. 21, no. 2, 1997, pages 111-117, XP009048966 ISSN: 0361-090X
- DATABASE WPI Section Ch, Week 199016 Derwent Publications Ltd., London, GB; Class A96, AN 1990-122399 XP002331704 & SU 1 512 955 A (UKR REFRACTORY IND) 7 October 1989 (1989-10-07)
- DATABASE MEDLINE [Online] US NATIONAL LIBRARY OF MEDICINE (NLM), BETHESDA, MD, US; September 1999 (1999-09), KAYAHARA H ET AL: "Synthesis and biological activities of ferulic acid derivatives." XP002331769 Database accession no. NLM10625955 & ANTICANCER RESEARCH. 1999 SEP-OCT, vol. 19, no. 5A, September 1999 (1999-09), pages 3763-3768, ISSN: 0250-7005
- SORIMACHI K.: 'Differential responses of lignin derivatives between tumor and normal tissue derived cell lines' CELL BIOLOGY INTERNATIONAL REPORTS vol. 16, no. 3, March 1992, pages 249 - 257, XP002905710

## Description

### TECHNICAL FIELD

This invention is related to compositions comprising lignins or lignin derivatives, and more particularly, to coatings for medical devices and the use of lignin in the manufacture of a medicament for inhibiting restenosis and thrombosis.

### BACKGROUND ART

Lignins are derived from an abundant and renewable resource: trees, plants, and agricultural crops. Commercial lignin is currently produced as a co-product of the paper industry, separated from trees by a chemical pulping process.

Industry first began to use lignins in the 1880s when lignosulfonates were used in leather tanning and dye baths. Since then, lignins have found applications in food products, serving as emulsifiers in animal feed and as raw material in the production of vanillin. Lignin uses have expanded and it is currently generally used as a binder, dispersant, emulsifier, or sequestrant (for complexing).

While lignins are known in the art they have not been widely used in the biomedical arena. Provided herein for the first time are bioactive activities of lignins and methods of making and using compositions comprising lignins having bioactive activities, particularly, for example, for use in the inhibition of cellular proliferation.

### DISCLOSURE OF INVENTION

In one embodiment, the invention relates to the use of lignin in the manufacture of a medicament for inhibiting restenosis.

In another embodiment, the invention relates to the use of lignin in the manufacture of a medicament for the inhibition of thrombosis.

In another embodiment, the invention relates to a coated medical device, wherein the coating comprises lignin.

In another embodiment, the invention relates to a method of forming a medica! device, said method comprising the step of coating a medical device, wherein the coating comprises lignin.

In the above embodiments, the preferred lignin is lignosulfonate.

### BRIEF DESCRIPTION OF THE DRAWINGS

FIG. 1 shows human smooth muscle cells, more specifically normal human smooth muscle cells as a control;
FIG. 2 shows normal human smooth muscle cells with 2.5% lignosulfate wherein no growth is indicated;
FIG. 3 shows human long fibroblast carcinoma cells, more particularly long fibroblast carcinoma cells as a control having 40% monolayer and 60% tumor cells;
FIG. 4 shows long fibroblast carcinoma cells treated with lignosulfate having 20% tumor cells and 40% monolayer; -
FIG. 5 shows the results of an ANOVA used to evaluate the statistical significance of raw data collected in an *in vivo* study of the effects of L3 on tumor cell development;
FIG. 6 is a scatter diagram showing tumor growth measurements made of mice that received injections of a 7.5% L3 solution in the *in vivo* study of the effects of L3 on tumor cell development; and
FIG. 7 is a scatter diagram showing tumor growth measurements of control mice in the *in vivo* study referred to in the brief description of FIGS. 5 and 6.

### BEST MODE FOR CARRYING OUT THE INVENTION

As further described below, it has been discovered herein that lignins and derivatives thereof have a number of bioactivities useful in the biomedical arena. Accordingly, provided herein are compositions comprising lignin or a derivative thereof and methods of making and using the same.

The compositions provided include coatings for medical devices, medical devices with coatings as described herein.

Lignins include lignosulfonates, also called lignin sulfonates and sulfite lignins, and Kraft lignins, also called sulfate lignins. Lignosulfonates are products of sulfite pulping and are hydrophilic. Kraft lignins are obtained from the Kraft pulping process and are hydrophobic. Other delignification technologies use an organic solvent or a high pressure steam treatment to remove lignins from plants are used as is known in the art. In one embodiment, lignosulfonates are used. In another embodiment, Kraft lignins are used.

Commercial lignin is currently produced as a co-product of the paper industry, separated from trees by a chemical pulping process. Generally, trees become logs and bark, all of which goes into chip silos and then through pulping, neutralizing, and evaporating processes, and then to a processing lignin plant where lignin products are produced. Lignins are produced by a variety of companies such as Lenox Polymers Ltd., based in Port Huron, Michigan, USA, Tembec, Inc. in Teemiscaming, Quebec, Lignotech, and Georgia Pacific.

In one embodiment, lignosulfonate is prepared by a conventional ethanol precipitation method from a commercially concentrated waste liquor (about 50% of solids concentration) such as Copartin TM. Briefly, Copartin TM can be diluted with a 5-fold excess of water, for example, and the solution added dropwise into a 10-fold excess of ethanol (for example). The precipitate is collected by centrifugation and then freeze-dried for further use. In one embodiment, the sugars are removed, for example, by hydrolyzing with sulfuric acid. This method is generally used for sugar composition analysis. Generally, 25 mg of lignosulfonate are dissolved in 0.5 ml of 72% (w/w) H₂SO₄ solution and digested at 30° C for an hour. After addition of 19.5 ml of water to the mixture it can be autoclaved and dialyzed using standard techniques to obtain sugar free lignosulfonate.

In one embodiment, the lignins are obtained from natural resources. The natural lignins may be modified to include or contain metals, halogens, salts, preferably divalent, or ammonium. Lignins containing iron are a preferred embodiment and have increased activity. Halogens include chlorine, bromine, fluorine, etc., though chlorine is preferred. Chlorinated lignins may be used in certain embodiments in which toxicity is not a factor, such as when diseased cells are targeted. In preferred embodiments, the lignins have minimal toxicity.

In another embodiment, the lignins are synthetic. For example, synthetic lignins are dehydrogenation polymers of p-coumaric acid, ferulic acid, and caffeic acid.

Generally, the lignins, natural or synthetic have increased activity when treated with a reducing agent such as NaBH₄. If desired, the activity can be decreased by treatment with an oxidizing agent such as ceric ammonium nitrate. When a lignin, natural or synthetic is manipulated to have constituents such as calcium, iron, etc., which are not part of the lignin as extracted or synthesized, the lignin is said to be a lignin-based agent or a derivative thereof. Lignin-derivatives as used herein are functional derivatives in that they retain lignin bioactivities as described herein, and preferably have an improved or, for some reason, desirable quality over the lignin on which the lignin derivative is based on. For example, a certain R group may be replaced on a lignin to increase or adjust specificity, activity, pH optimum, stability , etc.

The lignins or derivatives thereof as used herein are generally 1000-20,000 daltons (D), and preferably are about 8,000 to 15,000, more preferably about 9,000 to 12,000 D, and more preferably about 10,000 D. Generally, higher molecular weight lignins correlate with a purer product, which is preferred.

In preferred embodiments, the lignins or derivatives thereof have one or more bioactivities. In a preferred embodiment, the lignins used herein have anti-cellular proliferation activity. "Anti-cellular proliferation activity" as used herein is used interchangeably with "inhibition of cellular proliferation activity." Anti-cellular proliferation activity has a number of applications. For use in conjunction with medical devices used *in vivo*, anti-cellular proliferation activity inhibits or prevents tissue response reactions to inserted and/or implanted medical devices, which reactions result in tissue build up that can lead to disorders such as restenosis, scarring, rejection of the implant, etc.

In another embodiment, the lignins and derivatives thereof have "anti-thrombogenic activity," which, as used herein, is interchangeable with "inhibition of thrombus formation activity." In yet another embodiment, the lignins and derivatives thereof have "anti-restenosis activity."

The inhibitory or "anti-" activity of lignins as used herein, preferably inhibits the particular biological activity by any detectable amount, and more preferably by at least 30%, more preferably 40%, more preferably 50%, more preferably 70%, more preferably 90%, and most preferably by at least 95%. In one embodiment herein, inhibition is 100%. In one aspect, inhibition is defined as any detectable decrease in a particular biological activity, such as cellular proliferation, restenosis or thrombus formation compared to a control not comprising the lignin-containing composition.

In one aspect of the invention, a coating is provided, wherein the coating comprises lignin or a functional derivative thereof. In a preferred embodiment, a medical device is provided wherein the medical device has a coating thereon comprising lignin or a functional derivative thereof. The medical devices provided are preferably for use *in vivo.* The medical devices can be for limited use *in vivo*, such as operating or medical device tools such as catheters, laparoscopes, etc., or for temporary or permanent implant devices. In preferred embodiments, the device is selected from the group consisting of sutures, bone screws, nails, plates, tubes, sheets, films, stents, artificial valves and vessels, intra-aortic balloons, and prosthetics. In another embodiment, a medical device is defined as any material which may have tissue contact. In another embodiment, the medical device may be used for cell screening prior to injection. Moreover, the device may be coated, contain, be composed of, or manufactured from lignin or a derivative thereof.

The coating can be applied to the medical device by covalent or ionic binding to the surface of the medical device. Preferably, the coating is covalently bound to part or all of the surface of the medical device. The lignin can be applied as the coating directly or indirectly. Preferably, the lignin is applied indirectly by first mixing said lignin with a secondary coating, and then applying the secondary coating comprising lignin onto said medical device. The secondary coating preferably allows slow release of the lignins and can be any standard secondary coating such as those used to apply heparin to medical devices, generally comprising coated marbles. Usually, the percentage in a lignin coating is about 10% to 65%, more preferably about 20% to 60%, more preferably about 30% to 50%, and more preferably, the coating is about 30% lignin.

The coatings preferably contain lignin as the active agent and are exclusive of other active agents. In another embodiment, the coatings may contain one or more active agents in addition to lignin. For example, other anti-thrombotic agents may be added such as heparin. The coating can be applied by dipping, spraying, painting, electrostatic spraying, or vapor deposition, and is preferably dried at room temperature at 40 to 60% humidity. The coated device is then generally sterilized. The medical devices with the coatings thereon can then be used for their intended use as well as for the bioactivity of the lignin. Thus, reduction of tissue response to medical devices such as implants are also provided. For example, in one embodiment, inhibition of restenosis associated with the tissue response usually caused by a medical device is provided by use of a medical device having a coating comprising lignin. In another embodiment, inhibition of thrombosis associated with the tissue response usually caused by a medical device is provided by use of a medical device having a coating comprising lignin.

Disease states which can be treated by the uses provided herein include restenosis and thrombosis. Generally, the medical devices described herein are utilized to treat said disorders.

The individual, or patient, is generally a human subject, although as will be appreciated by those in the art, the patient may be animal as well. Thus other animals, including mammas such as rodents (including mice, rats, hamsters and guinea pigs), cats, dogs, rabbits, farm animals including cows, horses, goats, sheep, pigs, etc., and primates (including monkeys, chimpanzees, orangutans and gorillas) are included within the definition of patient. In a preferred embodiment, the individual requires inhibition of thrombus formation.

The medicaments for use as provided herein may be administered in a physiologically or pharmaceutically acceptable carrier to a host. The agents and compositions may be administered in a variety of ways, orally, systemically, topically, parenterally e.g., subcutaneously, intraperitoneally, intravascularly, etc. Depending upon the manner of introduction, the compounds may be formulated in a variety of ways. The concentration of therapeutically active compound in the formulation may vary from about 0.1-100 wt. %. Generally, a therapeutic amount for the need is used, for example, to achieve anti-thrombogenic activity.

Medicaments are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or PEG.

### EXAMPLES

### EXAMPLE 1: LIGNINS Inhibit Cell Growth In Vitro

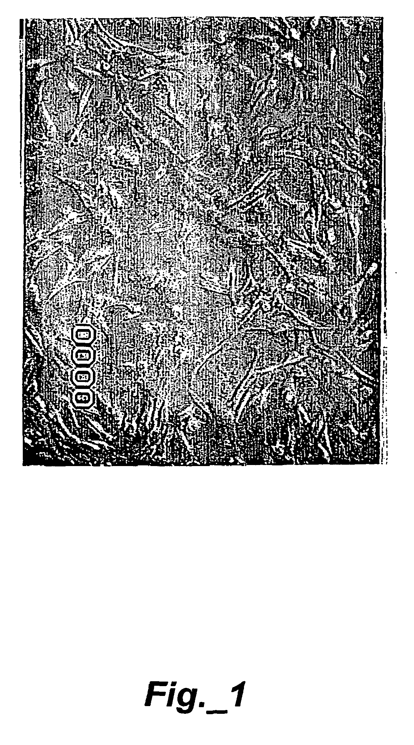

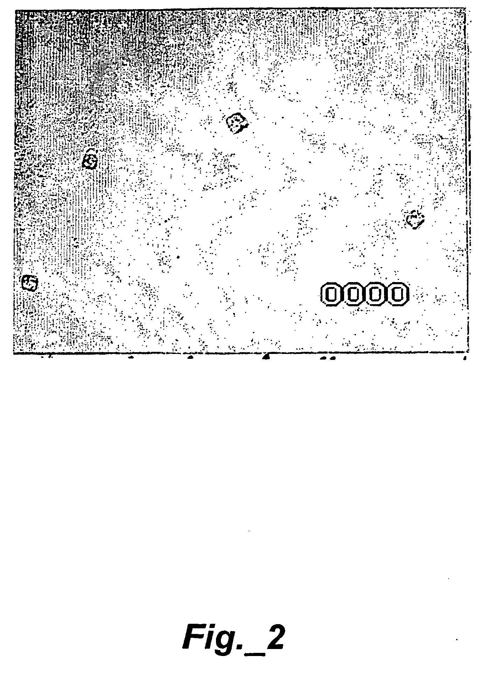

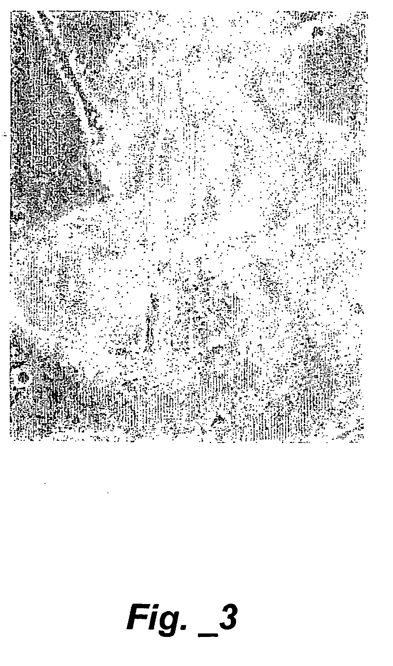

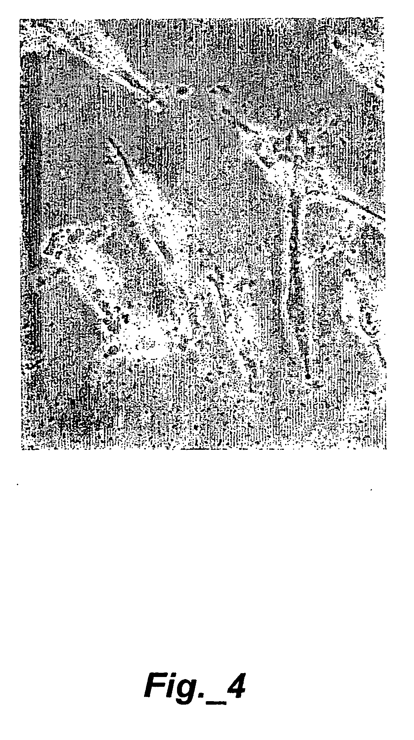

Lignosulfonate was added to human muscle cells in vitro. A 2.5% concentration of lignosulfate was added to the cells which were then incubated for an hour at 37 degrees C. The cells were then harvested and cultured for 20 hours. Cell growth was determined by observing whether cells adhered to the plate and continued to grow and divide. The results indicated that growth in human muscle cells is completely inhibited *in vitro* when exposed to lignosulfonate. Cancer cells showed similar results. See FIGS 1-4. Controls were maintained to ensure the integrity of the fat.

As also indicated in FIGS. 1-4, no sign of toxicity was observed. The toxicity was evaluated per ISO 10993 and USP 24. Mouse L929 fibroblast cells were grown in a culture. Once 80%-100% monolayer was achieved, the cells were subjected to 24 and 48 hour exposure to 2.5%, 5% and 10% lignosulfate. The cultures showed no sign of toxicity as stated by USP and ISO. Once again, controls were maintained to ensure the integrity of the fat.

### COMPARATIVE EXAMPLE 2: Lignins Have Anti-Microbial Activity

Lignosulfonate was added to a variety of microorganisms. The microorganisms were streaked directly onto a media (TSA) containing 2.5% and 5% lignosulfate. Negative control and positive controls were designed to ensure the integrity of the test method. The results indicate that growth was inhibited in *Streptomycetes epidermis, Aspergillus niger*, and *Penicillium spp*. The lignosulfonate did not appear to have a noticeable change on *Escherichia coli*. The inhibition is defined as delay or complete absence of microorganisms to grow on TSA plate containing lignosulfonate.

### COMPARATIVE EXAMPLE 3: Lignins Inhibit Tumor Cell Development In Vivo

Seventeen mice were received at the age of eight weeks. The mice were maintained and observed for approximately two weeks. Concurrently, melanoma cells specific for C57 mice were maintained and propagated to proper cell cultures. Two 25cm flasks were used to obtain an appropriate concentration of melanoma cells for injection. A flask containing 24-hour monolayer of cells was removed from the incubator. The monolayer was rinsed with 5ml of phosphate buffered saline and removed. 3ml of trypsin was added to the flask and placed in the incubator for 15 minutes ± 1 minute to allow cells to detach. 5ml of DMEM was added to the flask and pipetted down the flask wall to remove all cells. Total volume was brought up to 10mls with DMEM. The entire volume was transferred to a 50ml centrifuge tube. The volume of the second flask was transferred to the same tube. The cells were centrifuged for 10 minutes at 125xg. Old supernatant was removed and 10mls of fresh DMEM was added. A cell count was performed and the cells were spun down again. Supernatant was removed and the cells were suspended in sterile phosphate buffered saline and placed on ice for transport.

Mice were dropped into ajar containing ether soaked cotton balls. Upon sedation (approximately 20 seconds) mice were removed and injected with 0.050ml of cell suspension. Injections were intradermal on the right flank side. It was noted that the needle pierced through the skin to the outside on two of the injections; accordingly, the needle was withdrawn and the injection site moved to another location. Two mice were not injected and their ears punched with a single hole on the right side. They were maintained as negative controls.

Treatment with L3 began upon confirmation of tumor growth. Once tumors were confirmed, mice were separated into three groups of five. One group of five was maintained as a positive control and had no holes punched in their ears. Another group of five was designated with one hole in the right ear and received treatments with 5% L3. The final group of five mice were designated with a hole in each ear and received treatment with 7.5% L3. Treatment was administered every four days. L3 was prepared and sterilized immediately prior to injections. A new batch was made for every treatment. 0.020 ml of L3 was delivered to the tumor site using a sterile Hamilton syringe. Tumor size was measured using a dissecting ruler. Animals were laid down with their legs outstretched. Tumors were measured along the broadest dimension portion and the diameter of the tumor was recorded in centimeters. Overall health was documented prior to each treatment. Positive control animals were also measured and observed. All observations were documented on technician notes and maintained within the test folder.

Upon completion of treatment, mice were euthanized. One mouse from each test and control group was sampled for histology. Necroscopy was performed on all mice and observations documented.

### Results

Mice were confirmed to have a 93.33% uptake of tumors prior to treatment. A single mouse without a tumor was noted and designated null, but this same animal demonstrated a tumor present two days later and was placed in the positive control group.

Positive control mice began dying two days prior to the completion of the study. Tumors were extremely large and systemic infection was noted upon necroscopy. Livers were yellow, spleen was twice the negative size and pancreas were black. Two animals had hair loss on the neck and back and underside of appendages.

Four of the 5% test group died prior to completion of the study. Due to the loss of 5% animals throughout the study, only the 7.5% test group was evaluated statistically. The following results are macroscopic observations obtained throughout the study. Animal #4 died as a result of a relocation. Although there was tumor development, it was small and no infection had set in internally. Animal #4 died before the first treatment. Animal #5 had an open wound at the site of tumor growth as a result of piercing through the skin during delivery. Tumor site had an indentation that had constant bleeding. Treatment fluid leaked out of the wound. The probable cause of death was infection due to a decreased immune response. Animal #2 escaped from its cage and was found dead three days later. The apparent cause of death was dehydration and starvation. There was no discernable enlargement in the tumor and no sign of infection. Animal #1 was choked in its cage lid when another animal was removed for treatment. Its tumor was extremely small (comparable to a grain of sand) and its organs appeared normal. Animal #3 was euthanized at the end of the study. A second tumor was present and organs had a slight sign of infection.

In the 7.5% test group, one animal died prior to completion of the study. Animal #5 had an open wound at the site of cancer cell delivery. The tumor site had an indentation that had constant bleeding. Treatment fluid leaked out the wound. The probable cause of death was infection due to a decreased immune response. The remaining four animals had tumors that varied in size and various levels in internal infection.

The exposure of treatment schedule for mice included in the study is shown in Table I, below.

**TABLE I**

| Exposure and Treatment Schedule Chronology of *In Vivo* Study of the Effects of L3 on Tumor Cell Development | | |
|---|---|---|
| Date | Action | Comments |
| 6/27/2001 | Tumor Cell injection | 5.0x10⁵ cells/0.05ml |
| 6/29/2001 | Relocation | Sutter Medical, Sacramento |
| 7/9/2001 | 1^{st} treatment | 14/15 mice had confirmed tumors |
| 7/11/2001 | Mouse #4 - 5% died | Stressed from move |
| 7/13/2001 | 2^{nd} treatment | Two mice have open wounds |
| 7/13/2001 | Mouse #5 - 5% died | Due to open wound and infection |
| 7/16/2001 | Mouse #2 - 5% escaped | N/A |
| 7/17/2001 | 3^{rd} treatment | N/A |
| 7/19/2001 | Mouse #2 - 5% found | Found dead, lack of food and water |
| 7/19/2001 | Mouse #5 -7.5% died | Due to open wound and infection |
| 7/20/2001 | 4^{th} injection | N/A |
| 7/20/2001 | Mouse #1 - 5% died | Choked in cage lid, unable to inject |
| 7/20/2001 pm | Positive #3 died | Tumor ruptured, systemic infection |
| 7/23/2001 am | Positive #1 died | Tumor ruptured, systemic infection |
| 7/23/2001 | Necroscopy | Tumors and organs taken for histology |

### Analysis

Raw data for the study follows in Table II, below.

**TABLE II**

| Raw Data: Control Group vs. 7.5% Test Group | | | |
|---|---|---|---|
| Animal | Control | Test | |
| P-1 | 1.4 | 7.5-2 | 1.2 |
| P-1 | 2.6 | 7.5-2 | 1.4 |
| P-1 | 3 | 7.5-2 | 1.9 |
| P-1 | 3.1 | 7.5-2 | 2 |
| P-2 | 0.6 | 7.5-3 | 0.4 |
| P-2 | 1.9 | 7.5-3 | 0.7 |
| P-2 | 2.6 | 7.5-3 | 1.2 |
| P-2 | 2.8 | 7.5-3 | 1.6 |
| P-3 | 0.8 | | |
| P-3 | 1.8 | | |
| P-3 | 2.7 | | |
| P-3 | 2.4 | | |
| P-4 | 1.1 | | |
| P-4 | 2.4 | | |
| P-4 | 3 | | |
| P-4 | 3.1 | | |
| P-5 | 0.5 | | |
| P-5 | 1.7 | | |
| P-5 | 2 | | |
| P-5 | 2.1 | | |

Evaluation of the 7.5% test group was conducted. As per the Tumor Growth Table, Table III below, animal numbers 1 and 4 were not analyzed due to the inability to detect variance. Animal #5 was excluded from the analysis because it did not complete the study.

**TABLE III**

| Data and Observations Tumor Growth | | | | | |
|---|---|---|---|---|---|
| Treatment | 2^{nd} | 3^{rd} | 4^{th} | Terminate | % Increase |
| Animal | Size of tumor in cm | | | | |
| P-1 | 1.4 | 2.6 | 3.0 | 3.1 | 121 |
| P-2 | 0.6 | 1.9 | 2.6 | 2.8 | 367 |
| P-3 | 0.8 | 1.8 | 2.7 | 2.4 | 200 |
| P-4 | 1.1 | 2.4 | 3.0 | 3.1 | 181 |
| P-5 | 0.5 | 1.7 | 2.0 | 2.1 | 320 |
| 5-1 | SG | NC | NC | D | 0 |
| 5-2 | 0.7 | D | D | D | 0 |
| 5-3 | 1.2 | 1.4 | 1.9 | 2.1 | 75 |
| 5-4 | D | D | D | D | 0 |
| 5-5 | 0.8 | D | D | D | 0 |
| 7.5-1 | SG | NC | NC | SG | 0 |
| 7.5-2 | 1.2 | 1.4 | 1.9 | 2.0 | 67 |
| 7.5-3 | 0.4 | 0.7 | 1.2 | 1.6 | 300 |
| 7.5-4 | SG | 0.5 | 0.5 | 0.5 | 50 |
| 7.5-5 | 1.1 | 1.2 | D | D | 9 |

Key to Table III: SG = sand grain size, cannot be visualized or measured; D = animal removed from study due to death or escape; NC = no change in tumor size.

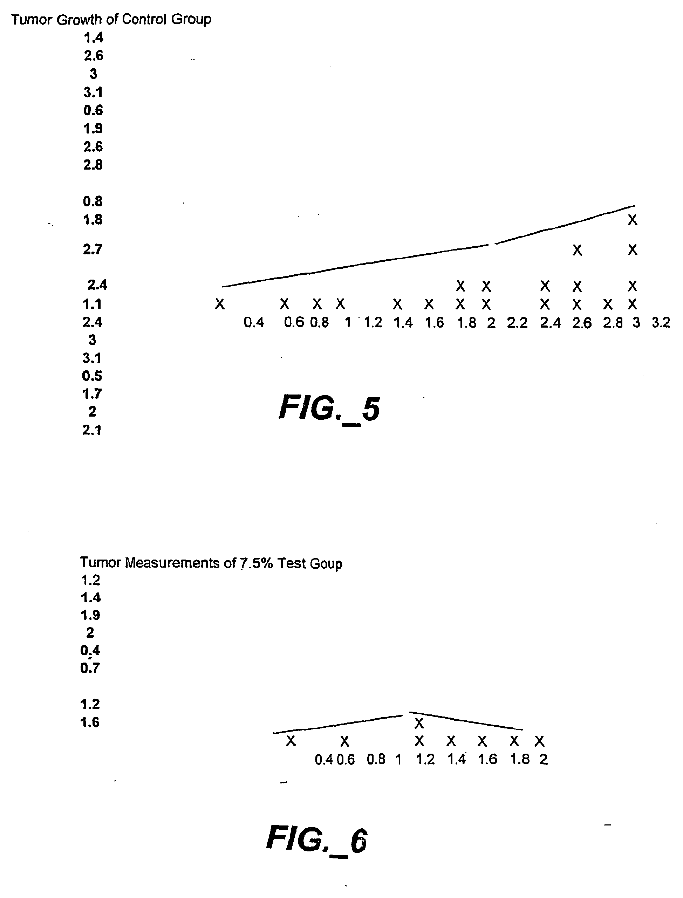

An ANOVA, represented in FIG. 5, was used to evaluate the statistical significance of the data. From the evaluation it may be concluded that L3 inhibits tumor development. This conclusion is based on a p value of 0.023362 and an assumed value of p=.05.

Animals numbered 1 and 4 within the 7.5% test group showed minimal growth when compared to results from control. The mean increase in tumor size in the control group was 238%. This compares to a mean tumor size increase in the 7.5% test group of 104%.

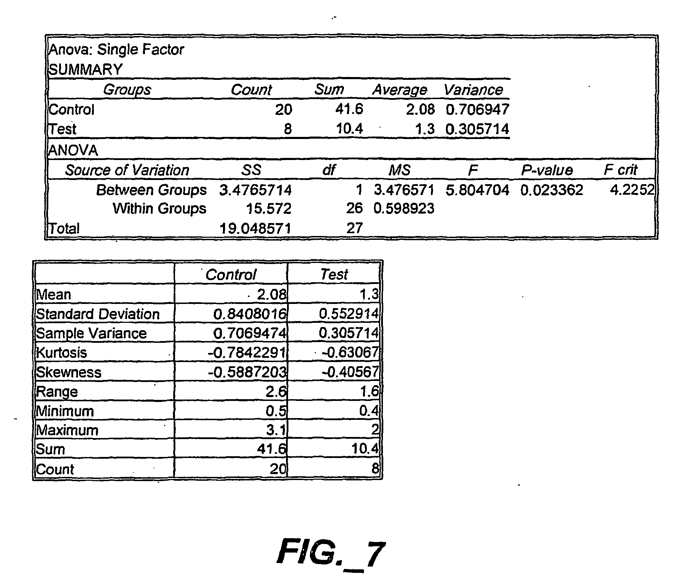

Upon review of scatter diagrams for the raw data, FIGS. 6-7, it is apparent that the tumor growth of the control group continued to increase while the test group demonstrated a limited growth. The control group showed a positive slope and the test group showed a negative slope. From the analysis it can be concluded that the 7.5% concentration of L3 has a reduction in cell proliferation when compared to control animals.

## Claims

1. Use of lignin in the manufacture of a medicament for inhibiting restenosis.

2. The use of claim 1, wherein the lignin is lignosulfonate.

3. The use of claim 2, wherein the lignosulfonate is prepared by ethanol precipitation from concentrated waste liquor.

4. The use of claim 2, wherein the lignosulfonate is obtained from natural resources.

5. The use of claim 1, wherein the lignin is synthetic lignin.

6. The use of claim 5, wherein the synthetic lignin is a dehydrogenation polymer of p-coumaric acid, ferulic acid, and caffeic acid.

7. The use of claim 5 or 6, wherein the lignin is treated with a reducing agent.

8. The use of claim 7, where in the reducing agent is NaBH₄.

9. The use of claim 5 or 6, wherein the lignin is treated with an oxidizing agent.

10. The use of claim 9, wherein the oxidizing agent is ceric ammonium nitrate.

11. The use of any one of claims 1 to 10, wherein the lignin is modified to contain iron.

12. The use of any one of claims 1 to 11, wherein the lignin has a molecular weight between 1,000 and 20,000 daltons, between 8,000 and 15,000 daltons or between 9,000 and 12,000 daltons.

13. The use of any one of claims 1 to 12, wherein the lignin is formulated for administration with a pharmaceutically acceptable carrier.

14. The use of any one of claims 1 to 13, where in the lignin is formulated with a pharmaceutically acceptable carrier and a ligand that targets diseased cells.

15. Use of lignin in the manufacture of a medicament for the inhibition of thrombosis formation.

16. The use of claim 15, wherein the lignin has the features of any one of claims 2 to 13.

17. A coated medical device, wherein the coating comprises lignin.

18. The device of claim 17, wherein the device is selected from the group consisting of sutures, bone screws, nails, plates, tubes, sheets, films, stents, artificial valves and vessels, intra-aortic balloons, and prosthetics.

19. The device of claim 17 or 18, wherein the lignin is lignosulfonate.

20. The device of claim 19, wherein the lignosulfonate is prepared by ethanol precipitation from concentrated waste liquor.

21. The device of claim 19, wherein the lignosulfonate is obtained from natural resources.

22. The device of claim 17 or 18, wherein the lignin is synthetic lignin.

23. The device of claim 22, wherein the synthetic lignin is a dehydrogenation polymer of p-coumaric acid, ferulic acid, and caffeic acid.

24. The device of claim 22 or 23, wherein the lignin is treated with a reducing agent.

25. The device of claim 23, wherein the reducing agent is NaBH₄.

26. The device of claim 22 or 23, wherein the lignin is treated with an oxidizing agent.

27. The device of claim 26, wherein the oxidizing agent is ceric ammonium nitrate.

28. The device of any one of claims 17 to 27, wherein the lignin is modified to contain iron.

29. The device of claim 28, wherein the lignin has a molecular weight between 1,000 and 20,000 daltons, between 8,000 and 15,000 daltons or between 9,000 and 12,000 daltons.

30. A method of forming a medical device, said method comprising the step of coating a medical device, wherein the coating comprises lignin.

31. The method of claim 30, wherein the lignin is lignosulfonate.

32. The method of claim 30 and 31, wherein the device is for use *in vivo*.

33. The method of any one of claims 30 to 32, wherein the device is selected from the group consisting of sutures, bone screws, nails, plates, tubes, sheets, films, stents, artificial valves and vessels, intra-aortic balloons, and prosthetics.

34. The method of any one of claims 30 to 33, wherein the coating is applied by dipping, spraying, painting, electrostatic spraying, or vapor deposition.

35. The method of any one of claims 30 to 34, further comprising the step of mixing the coating with a secondary coating before coating the medical device.

36. The method of claim 35, wherein the secondary coating allows the slow release of the coating when using the medical device *in vivo.*

37. The method of claim 35, where the secondary coating comprises coated marbles.

38. The method of claim 35, wherein the mixture of coating and secondary coating includes lignin in an amount between 10% and 65% by weight, or between 20% and 60% by weight or between 30% and 50% by weight or approximately 30% by weight.

## Patentansprüche

1. Verwendung von Lignin zur Herstellung eines Arzneimittels zur Hemmung von Restenose.

2. Verwendung nach Anspruch 1, wobei das Lignin Lignosulfonat ist.

3. Verwendung nach Anspruch 2, wobei das Lignosulfonat durch Ausfällung mit Ethanol aus konzentrierter Ablauge hergestellt wird.

4. Verwendung nach Anspruch 2, wobei das Lignosulfonat aus natürlichen Quellen erhalten wird.

5. Verwendung nach Anspruch 1, wobei das Lignin synthetisches Lignin ist.

6. Verwendung nach Anspruch 5, wobei das synthetische Lignin ein dehydriertes Polymer aus p-Cumarsäure, Ferulasäure und Kaffeesäure ist.

7. Verwendung nach Anspruch 5 oder 6, wobei das Lignin mit einem Reduktionsmittel behandelt wird.

8. Verwendung nach Anspruch 7, wobei das Reduktionsmittel NaBH₄ ist.

9. Verwendung nach Anspruch 5 oder 6, wobei das Lignin mit einem Oxidationsmittel behandelt wird.

10. Verwendung nach Anspruch 9, wobei das Oxidationsmittel Cerammoniumnitrat ist.

11. Verwendung nach einem der Ansprüche 1 bis 10, wobei das Lignin so modifiziert ist, dass es Eisen enthält.

12. Verwendung nach einem der Ansprüche 1 bis 11, wobei das Lignin ein Molekulargewicht zwischen 1000 und 20000 Dalton, zwischen 8000 und 15000 Dalton oder zwischen 9000 und 12000 Dalton aufweist.

13. Verwendung nach einem der Ansprüche 1 bis 12, wobei das Lignin zur Verabreichung mit einem pharmazeutisch annehmbaren Träger formuliert ist.

14. Verwendung nach einem der Ansprüche 1 bis 13, wobei das Lignin mit einem pharmazeutisch annehmbaren Träger und einem Liganden formuliert ist, der auf die erkrankten Zellen abzielt.

15. Verwendung von Lignin zur Herstellung eines Arzneimittels zur Hemmung von Thrombosebildung.

16. Verwendung nach Anspruch 15, wobei das Lignin die Eigenschaften eines der Ansprüche 2 bis 13 aufweist.

17. Beschichtetes Medizinprodukt, wobei die Beschichtung Lignin umfasst.

18. Produkt nach Anspruch 17, wobei das Produkt ausgewählt ist aus der Gruppe bestehend aus Wundnähten, Knochenschrauben, Nägeln, Platten, Rohren, Folien, Filmen, Stents, künstlichen Klappen und Gefäßen, intra-aortalen Ballons und Prothesen.

19. Produkt nach Anspruch 17 oder 18, wobei das Lignin Lignosulfonat ist.

20. Produkt nach Anspruch 19, wobei das Lignosulfonat durch Ausfällung mit Ethanol aus konzentrierter Ablauge hergestellt wird.

21. Produkt nach Anspruch 19, wobei das Lignosulfonat aus natürlichen Quellen erhalten wird.

22. Produkt nach Anspruch 17 oder 18, wobei das Lignin synthetisches Lignin ist.

23. Produkt nach Anspruch 22, wobei das synthetische Lignin ein dehydriertes Polymer aus p-Cumarsäure, Ferulasäure und Kaffeesäure ist.

24. Produkt nach Anspruch 22 oder 23, wobei das Lignin mit einem Reduktionsmittel behandelt wird.

25. Produkt nach Anspruch 23, wobei das Reduktionsmittel NaBH₄ ist.

26. Produkt nach Anspruch 22 oder 23, wobei das Lignin mit einem Oxidationsmittel behandelt wird.

27. Produkt nach Anspruch 26, wobei das Oxidationsmittel Cerammoniumnitrat ist.

28. Produkt nach einem der Ansprüche 17 bis 27, wobei das Lignin so modifiziert ist, dass es Eisen enthält.

29. Produkt nach Anspruch 28, wobei das Lignin ein Molekulargewicht zwischen 1000 und 20000 Dalton, zwischen 8000 und 15000 Dalton oder zwischen 9000 und 12000 Dalton aufweist.

30. Verfahren zum Herstellen eines Medizinproduktes, wobei das Verfahren den Schritt des Beschichtens eines Medizinproduktes umfasst, wobei die Beschichtung Lignin umfasst.

31. Verfahren nach Anspruch 30, wobei das Lignin Lignosulfonat ist.

32. Verfahren nach Anspruch 30 und 31, wobei das Produkt zur Verwendung *in vivo* vorgesehen ist.

33. Verfahren nach einem der Ansprüche 30 bis 32, wobei das Produkt ausgewählt ist aus der Gruppe bestehend aus Wundnähten, Knochenschrauben, Nägeln, Platten, Rohren, Folien, Filmen, Stents, künstlichen Klappen und Gefäßen, intra-aortalen Ballons und Prothesen.

34. Verfahren nach einem der Ansprüche 30 bis 33, wobei die Beschichtung durch Eintauchen, Besprühen, Lackieren, Elektrolackieren oder Aufdampfen aufgebracht wird.

35. Verfahren nach einem der Ansprüche 30 bis 34, weiterhin umfassend den Schritt des Mischens der Beschichtung mit einer zweiten Beschichtung, bevor das Medizinprodukt beschichtet wird.

36. Verfahren nach Anspruch 35, wobei die zweite Beschichtung die langsame Freisetzung der Beschichtung bei Verwendung des Medizinprodukts *in vivo* ermöglicht.

37. Verfahren nach Anspruch 35, wobei die zweite Beschichtung beschichtete Kügelchen umfasst.

38. Verfahren nach Anspruch 35, wobei das Gemisch aus der Beschichtung und der zweiten Beschichtung Lignin in einer Menge zwischen 10 Gew.-% und 65 Gew.-% oder zwischen 20 Gew.-% und 60 Gew.-% oder zwischen 30 Gew.-% und 50 Gew.-% oder etwa 30 Gew.-% enthält.

## Revendications

1. Utilisation de lignine dans la fabrication d'un médicament destiné à inhiber la resténose.

2. Utilisation selon la revendication 1, dans laquelle la lignine est un lignosulfonate.

3. Utilisation selon la revendication 2, dans laquelle le lignosulfonate est préparé par précipitation à l'éthanol à partir d'une liqueur résiduaire concentrée.

4. Utilisation selon la revendication 2, dans laquelle le lignosulfonate est obtenu à partir de ressources naturelles.

5. Utilisation selon la revendication 1, dans laquelle la lignine est une lignine synthétique.

6. Utilisation selon la revendication 5, dans laquelle la lignine synthétique est un polymère de déshydrogénation d'acide *p*-coumarique, d'acide férulique et d'acide caféique.

7. Utilisation selon la revendication 5 ou 6, dans laquelle la lignine est traitée avec un agent réducteur.

8. Utilisation selon la revendication 7, dans laquelle l'agent réducteur est NaBH₄.

9. Utilisation selon la revendication 5 ou 6, dans laquelle la lignine est traitée avec un agent oxydant.

10. Utilisation selon la revendication 9, dans laquelle l'agent oxydant est le nitrate cérique d'ammonium.

11. Utilisation selon l'une quelconque des revendications 1 à 10, dans laquelle la lignine est modifiée pour contenir du fer.

12. Utilisation selon l'une quelconque des revendications 1 à 11, dans laquelle la lignine a un poids moléculaire compris entre 1000 et 20 000 daltons, entre 8000 et 15 000 daltons ou entre 9000 et 12 000 daltons.

13. Utilisation selon l'une quelconque des revendications 1 à 12, dans laquelle la lignine est formulée pour l'administration avec un support pharmaceutiquement acceptable.

14. Utilisation selon l'une quelconque des revendications 1 à 13, dans laquelle la lignine est formulée avec un support pharmaceutiquement acceptable et un ligand qui cible des cellules malades.

15. Utilisation de lignine dans la fabrication d'un médicament destiné à inhiber la formation de thrombose.

16. Utilisation selon la revendication 15, dans laquelle la lignine a les caractéristiques de l'une quelconque des revendications 2 à 13.

17. Dispositif médical revêtu, dans lequel le revêtement comprend de la lignine.

18. Dispositif selon la revendication 17, dans lequel le dispositif est choisi dans le groupe formé par des sutures, vis à os, clous, plaques, tubes, feuilles, films, endoprothèses vasculaires, valvules et vaisseaux artificiels, ballonnets intra-aortiques et prothèses.

19. Dispositif selon la revendication 17 ou 18, dans lequel la lignine est un lignosulfonate.

20. Dispositif selon la revendication 19, dans lequel le lignosulfonate est préparé par précipitation à l'éthanol à partir d'une liqueur résiduaire concentrée.

21. Dispositif selon la revendication 19, dans lequel le lignosulfonate est obtenu à partir de ressources naturelles.

22. Dispositif selon la revendication 17 ou 18, dans lequel la lignine est une lignine synthétique.

23. Dispositif selon la revendication 22, dans lequel la lignine synthétique est un polymère de déshydrogénation d'acide *p*-coumarique, d'acide férulique et d'acide caféique.

24. Dispositif selon la revendication 22 ou 23, dans lequel la lignine est traitée avec un agent réducteur.

25. Dispositif selon la revendication 23, dans lequel l'agent réducteur est NaBH₄.

26. Dispositif selon la revendication 22 ou 23, dans lequel la lignine est traitée avec un agent oxydant.

27. Dispositif selon la revendication 26, dans lequel l'agent oxydant est le nitrate cérique d'ammonium.

28. Dispositif selon l'une quelconque des revendications 17 à 27, dans lequel la lignine est modifiée pour contenir du fer.

29. Dispositif selon la revendication 28, dans lequel la lignine a un poids moléculaire compris entre 1000 et 20 000 daltons, entre 8000 et 15 000 daltons ou entre 9000 et 12 000 daltons.

30. Procédé de formation d'un dispositif médical, ledit procédé comprenant l'étape consistant à revêtir un dispositif médical, dans lequel le revêtement comprend de la lignine.

31. Procédé selon la revendication 30, dans lequel la lignine est un lignosulfonate.

32. Procédé selon les revendications 30 et 31, dans lequel le dispositif est destiné à être utilisé *in vivo.*

33. Procédé selon l'une quelconque des revendications 30 à 32, dans lequel le dispositif est choisi dans le groupe formé par des sutures, vis à os, clous, plaques, tubes, feuilles, films, endoprothèses vasculaires, valvules et vaisseaux artificiels, ballonnets intra-aortiques et prothèses.

34. Procédé selon l'une quelconque des revendications 30 à 33, dans lequel le revêtement est appliqué par immersion, pulvérisation, application de peinture, pulvérisation électrostatique ou dépôt en phase vapeur.

35. Procédé selon l'une quelconque des revendications 30 à 34, comprenant de plus l'étape consistant à mélanger le revêtement avec un revêtement secondaire avant de revêtir le dispositif médical.

36. Procédé selon la revendication 35, dans lequel le revêtement secondaire permet la libération lente du revêtement lorsque le dispositif médical est utilisé *in vivo.*

37. Procédé selon la revendication 35, dans lequel le revêtement secondaire comprend des billes enrobées.

38. Procédé selon la revendication 35, dans lequel le mélange de revêtement et de revêtement secondaire comprend de la lignine en une quantité comprise entre 10 % et 65 % en poids, ou entre 20 % et 60 % en poids, ou entre 30 % et 50 % en poids, ou d'environ 30 % en poids.
